# EUROPEAN PATENT SPECIFICATION

(11) **EP 3 576 837 B1**
(45) Date of publication and mention of the grant of the patent: **16.09.2026**
(21) Application number: 18704775.8
(22) Date of filing: 31.01.2018
(51) Int. Cl.: A61N 1/36, A61N 1/372

(54) **STIMULATION FIELD TEMPLATES TO BE APPLIED ACROSS PATIENT POPULATIONS**
ÜBER PATIENTENPOPULATIONEN HINWEG ANZUWENDENDE STIMULATIONSFELDVORLAGEN
MODÈLES DE CHAMP DE STIMULATION À APPLIQUER SUR DES POPULATIONS DE PATIENTS

(30) Priority: 01.02.2017 US 201762453052 P
(43) Date of publication of application: 11.12.2019
(73) Proprietor: Boston Scientific Neuromodulation Corporation, Valencia, CA 91355 (US)
(72) Inventor: KOTHANDARAMAN, Sridhar, Valencia, CA 91355 (US); KASHYAP, Dheerendra Raghavendra, Valencia, CA 91354 (US); VANSICKLE, Dennis, Allen, Lancaster, CA 93536 (US); SHAH, Chirag, Valencia, CA 91354 (US)
(74) Representative: Vossius & Partner Patentanwälte Rechtsanwälte mbB
(86) International application number: PCT/US2018/016110
(87) International publication number: WO 2018/144521

(56) References cited:
- EP-A1- 2 567 731
- US-A1- 2011 270 358
- US-A1- 2013 268 026
- US-A1- 2016 279 429

## Description

### TECHNICAL FIELD

This document relates generally to medical devices, and more particularly, but not by way of limitation, to systems, devices, and methods to deliver neural modulation.

### BACKGROUND

Neurostimulation, also referred to as neuromodulation, has been proposed as a therapy for a number of conditions. Examples of neurostimulation include Spinal Cord Stimulation (SCS), Deep Brain Stimulation (DBS), Peripheral Nerve Stimulation (PNS), and Functional Electrical Stimulation (FES). Implantable neurostimulation systems have been applied to deliver such a therapy. An implantable neurostimulation system may include an implantable neurostimulator, also referred to as an implantable pulse generator (IPG), and one or more implantable leads each including one or more electrodes. The implantable neurostimulator may deliver neurostimulation energy through one or more electrodes placed on or near a target site in the nervous system, and an external programming device may be used to program the implantable neurostimulator with stimulation parameters controlling the delivery of the neurostimulation energy. Neurostimulation energy may be delivered using electrical energy that may have stimulation parameters to specify spatial (where to stimulate), temporal (when to stimulate) and/or informational (stimulation patterns directing the nervous system to respond as desired) aspects of a pattern of a neurostimulation waveform.

EP 2 567 731 A1 discloses stimulation systems and and tools for programming an electrical stimulation generator

### SUMMARY

This document discusses, among other things, systems and methods to receive a target neuromodulation field location within a patient for a neuromodulation field, wherein the target neuromodulation field location is not specific to the patient, receive a waveform file, the waveform file including data to define a waveform morphology for a neuromodulation waveform, wherein the waveform morphology is not specific to the patient, using the waveform file and the target neuromodulation field location to determine a patient-specific neuromodulation waveform, and deliver the patient-specific neuromodulation waveform to the at least one electrode to provide a neuromodulation field at the target neurostimulation field location.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 illustrates an example of a neurostimulation system.
FIG. 2 illustrates an example of a stimulation device and a lead system, such as may be implemented in the neurostimulation system of FIG. 1.
FIG. 3 illustrates an example of a programming device, such as may be implemented in the neurostimulation system of FIG. 1.
FIG. 4 illustrates an example of an implantable neurostimulation system and portions of an environment in which the system may be used.
FIG. 5 illustrates an example of an implantable stimulator and one or more leads of an implantable neurostimulation system, such as the implantable system of FIG. 4.
FIG. 6 illustrates an example of an external programming device of an implantable neurostimulation system, such as the external system of FIG. 4.
FIG. 7 illustrates an example of a waveform composer of an external programming device, such as the external programming device of FIG. 6.
FIG. 8 illustrates an example of a waveform composer of an external programming device, such as the external programming device of FIG. 6.
FIG. 9A illustrates an example of a method of mapping of target electrical fields to electrodes.
FIG. 9B illustrates an example of a method of determining fractionalization to achieve an objective function.
FIG. 10 illustrates an example of a method of determining fractionalization to achieve an objective function with more detail.
FIG. 11A-11B illustrate examples of a method of mapping a target electrical field to an electrode array.
FIG. 12A-12C illustrate examples of a method of selection of a plurality of constituent current sources at the locations of the electrodes.
FIG. 13 illustrates an example of an m x n transfer matrix used to determine the relative strengths of constituent current sources.
FIG. 14A illustrates an example of a method for adapting a generic neuromodulation waveform to a specific patient.
FIG. 14B illustrates an example of a generic neuromodulation waveform.
FIG. 14C illustrates an example of a patient-specific neuromodulation waveform.
FIG. 15A illustrates an example of a method for providing a massage pattern to a patient.
FIG. 15B illustrates an example of an electrode configuration for providing neurostimulation to an initial target location.
FIG. 15C-15D illustrate examples of a time varying neurostimulation pattern.
FIG. 16A-16C illustrate examples of stimulation patterns.
FIG. 17 illustrates a block diagram of an example machine upon which any one or more of the techniques (e.g., methodologies) discussed herein may perform.

### DETAILED DESCRIPTION

Clinically, a system and method are provided for adapting a generic neuromodulation waveform to a specific patient, such as to provide pain relief, deep brain stimulation, peripheral nerve stimulation, vagal nerve stimulation, or motor control therapy to the patient.

In certain systems, a neuromodulation waveform that provides pain relief to a patient, may not provide pain relief to other patients in the absence of adjustments to the neuromodulation waveform. The present inventors have recognized, among other things, the desirability to use a generic waveform, and then adapt the generic waveform to specific patients, such as to provide pain relief to the patients.

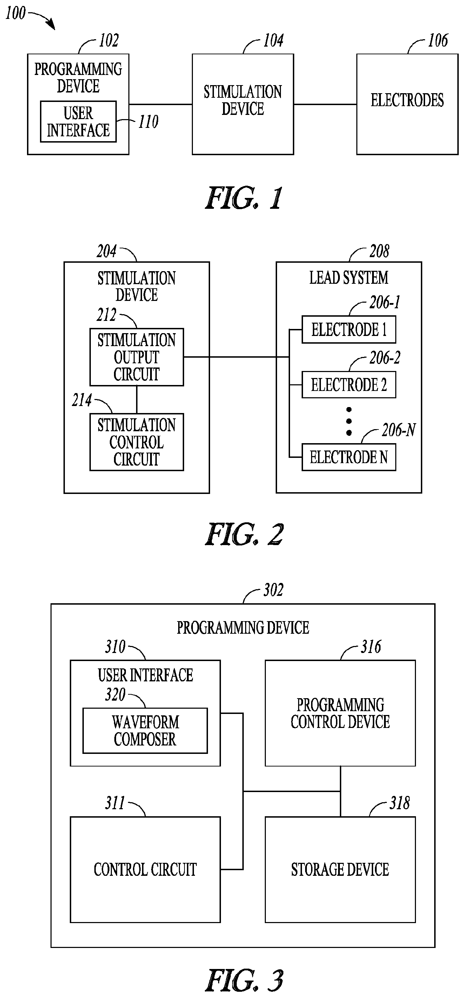

FIG. 1 illustrates an example of a neurostimulation system 100. The neurostimulation system 100 may include electrodes 106, a stimulation device 104, and a programming device 102. The electrodes 106 may be configured to be placed on or near one or more neural targets in a patient. The stimulation device 104 may be configured to be electrically connected to the electrodes 106 and deliver neurostimulation energy, such as in the form of an electrical waveform, to the one or more neural targets though the electrodes 106. The delivery of the neurostimulation may be controlled using a plurality of stimulation parameters, such as stimulation parameters specifying a waveform shape or waveform morphology such as, but not limited to, a pattern of electrical pulses and a selection of electrodes through which each of the electrical pulses may be delivered. At least some parameters of the plurality of stimulation parameters may be programmable by a user, such as a physician or other caregiver who treats the patient using the neurostimulation system 100. Programming device 102 may provide the user with accessibility to the user-programmable parameters. The programming device 102 may be configured to be communicatively coupled to stimulation device 104 via a wired or wireless link. The programming device 102 may receive a signal from the patient and based on the received signal, the programming device 102 may automatically adjust the stimulation parameters, such as to provide improved pain relief to the patient. The received signal may include information about a sensitivity of the patient to delivered neurostimulation energy. In an example where the electrodes may be implanted in the patient, the received signal may include information about the position of the electrodes 106 within the patient.

In an example, the programming device 102 may include a user interface that allows the user to set and/or adjust values of the user-programmable parameters by creating and/or editing graphical representations of various waveforms. Such waveforms may include different waveform shapes. The waveform shapes may include regular shapes (e.g. square, sinusoidal, triangular, saw tooth, and the like) or irregular shapes. Such waveforms may include, for example, a pattern of neurostimulation pulses to be delivered to the patient as well as waveform building blocks that can be used in the pattern of neurostimulation pulses. Examples of such waveform building blocks may include pulses, bursts each including a group of the pulses, trains each including a group of the bursts, and sequences each including a group of the pulses, bursts, and trains, as further discussed below. In various embodiments, programming device 102 allows the user to edit existing waveform building blocks, create new waveform building blocks, import waveform building blocks created by other users, and/or export waveform building blocks to be used by other users. The user may also be allowed to define an electrode selection specific to each waveform building block. In the illustrated embodiment, the user interface may include a user interface 110. In various embodiments, the user interface 110 may include a GUI or any other type of user interface accommodating various functions including waveform composition as discussed in this document. In an example, the programming device 102 may receive a waveform file. The waveform file may include a waveform shape or a sequence of waveform building blocks. In an example, the programming device may receive a target location for the neurostimulation energy. The neurostimulation system 100 may deliver an electrical waveform to the received target location, and the electrical waveform may have a shape according to a received waveform file.

FIG. 2 illustrates an example of a stimulation device 204 and a lead system 208, such as may be implemented in the neurostimulation system 100. The stimulation device 204 may represent an example of the stimulation device 104 and may include a stimulation output circuit 212 and a stimulation control circuit 214. The stimulation output circuit 212 may produce and deliver a neurostimulation waveform. Such waveforms may include different waveform shapes. The waveform shapes may include regular shapes (e.g. square, sinusoidal, triangular, saw tooth, and the like) or irregular shapes. The stimulation control circuit 214 may control the delivery of the neurostimulation waveform using the plurality of stimulation parameters, which specifies a pattern of the neurostimulation waveform. The lead system 208 may include one or more leads each configured to be electrically connected to stimulation device 204 and a plurality of electrodes 206 distributed in the one or more leads. The plurality of electrodes 206 may include electrode 206-1, electrode 206-2, ... electrode 206-N, each a single electrically conductive contact providing for an electrical interface between the stimulation output circuit 212 and the tissue of the patient, where N ≥ 2. The neurostimulation waveform may be delivered from stimulation output circuit 212 through a set of electrodes selected from electrodes 206.

In an example, the number of leads and the number of electrodes on each lead depend on, for example, the distribution of target(s) of the neurostimulation and the need for controlling the distribution of electric field at each target. In an example, the lead system 208 includes 2 leads each having 8 electrodes.

FIG. 3 illustrates an example of a programming device 302, such as may be implemented in the neurostimulation system 100. The programming device 302 may represent an embodiment of the programming device 102 and may include a storage device 318, a programming control circuit 316, a control circuit 311 and a user interface 310. The storage device 318 may store a plurality of waveform building blocks. The programming control circuit 316 may generate a plurality of stimulation parameters that control the delivery of the neurostimulation waveform according to the pattern of the neurostimulation waveform. The control circuit 311 may receive a signal and may adjust the values of the plurality of stimulation parameters based on the received signal. The received signal may include information about a patient sensitivity to delivered neurostimulation (e.g., information about an intensity or location of the delivered neurostimulation). The control circuit 311 may determine at least one stimulation parameter based on the information about the patient sensitivity. The received signal may include information about a position of an electrode relative to the patient. The electrode may be an implanted electrode within the patient or may be external to the patient. The control circuit 311 may determine at least one stimulation parameter based on the position of the electrode relative to the patient. The user interface 310 may represent an embodiment of the user interface 110 and allow the user to compose the waveform building blocks and compose the pattern of the neurostimulation waveform using one or more waveform building blocks selected from the plurality of waveform building blocks.

In an example, the user interface 310 may include a waveform composer 320 that allows the user to manage the waveform building blocks, including creating and importing waveform building blocks to be added to the waveform building blocks stored in storage device 318, exporting waveform building blocks selected from the waveform building blocks stored in storage device 318, and editing each of the waveform building blocks. In an example, the user interface 310 may include a GUI that allows for graphical editing of each of the waveform building blocks. In an example, the waveform composer 320 may allow the user to compose the pattern of the neurostimulation waveform to be delivered to the patent by the stimulation device 104 using waveform building blocks such as, but not limited to pulses, bursts each including a group of the pulses, trains each including a group of the bursts, and/or sequences each including a group of the pulses, bursts, and trains. In an example, the waveform composer 320 may allow the user to create each waveform building block using one or more waveform building blocks stored in the storage device 318 as templates. In an example, the waveform composer 320 may allow each newly created waveform building block to be saved as an additional waveform building block stored in the storage device 318.

In an example, the user interface 310 may include, but is not limited to, a touchscreen. In an example, the user interface 310 may include any type of presentation device, such as interactive or non-interactive screens, and any type of user input devices that allow the user to edit the waveforms or building blocks and schedule the programs, such as touchscreen, keyboard, keypad, touchpad, trackball, joystick, and mouse. In an example, the circuits of neurostimulation system 100, including its various embodiments discussed in this document, may be implemented using a combination of hardware and software. For example, the circuit of the user interface 110, the stimulation control circuit 214, and the programming control circuit 316, including their various embodiments discussed in this document, may be implemented using an application-specific circuit constructed to perform one or more particular functions or a general-purpose circuit programmed to perform such function(s). Such a general-purpose circuit may include, but is not limited to, a microprocessor or a portion thereof, a microcontroller or portions thereof, and a programmable logic circuit or a portion thereof.

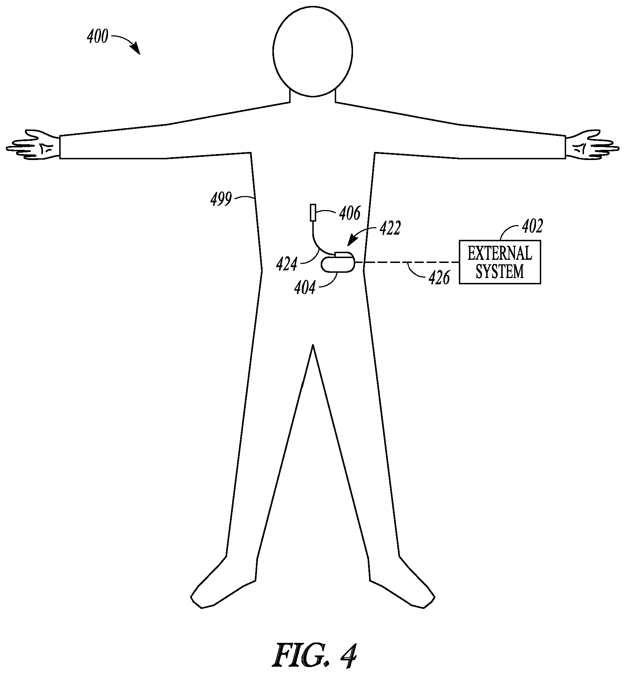

FIG. 4 illustrates, by way of example and not limitation, an implantable neurostimulation system 400 and portions of an environment in which system 400 may be used. The system 400 may include an implantable system 422, an external system 402, and a telemetry link 426 providing for wireless communication between implantable system 422 and external system 402. The implantable system 422 is illustrated in FIG. 4 as being implanted in the patient's body 499.

The implantable system 422 may include an implantable stimulator (also referred to as an implantable pulse generator, or IPG) 404, a lead system 424, and electrodes 406, which may represent an embodiment of stimulation device 204, lead system 208, and electrodes 206, respectively. The external system 402 may represent an embodiment of programming device 302. In an example, the external system 402 includes one or more external (non-implantable) devices each allowing the user and/or the patient to communicate with implantable system 422. In an example, the external system 402 may include a programming device intended for the user to initialize and adjust settings for the implantable stimulator 404 and a remote control device intended for use by the patient. For example, the remote control device may allow the patient to turn the implantable stimulator 404 on and off and/or adjust certain patient-programmable parameters of the plurality of stimulation parameters.

The sizes and shapes of the elements of the implantable system 422 and their location in the body 499 are illustrated by way of example and not by way of restriction. In various examples, the present subject matter may be applied in programming any type of stimulation device that uses electrical waveforms or electrical pulses as stimuli, regardless of stimulation targets in the patient's body and whether the stimulation device is implantable.

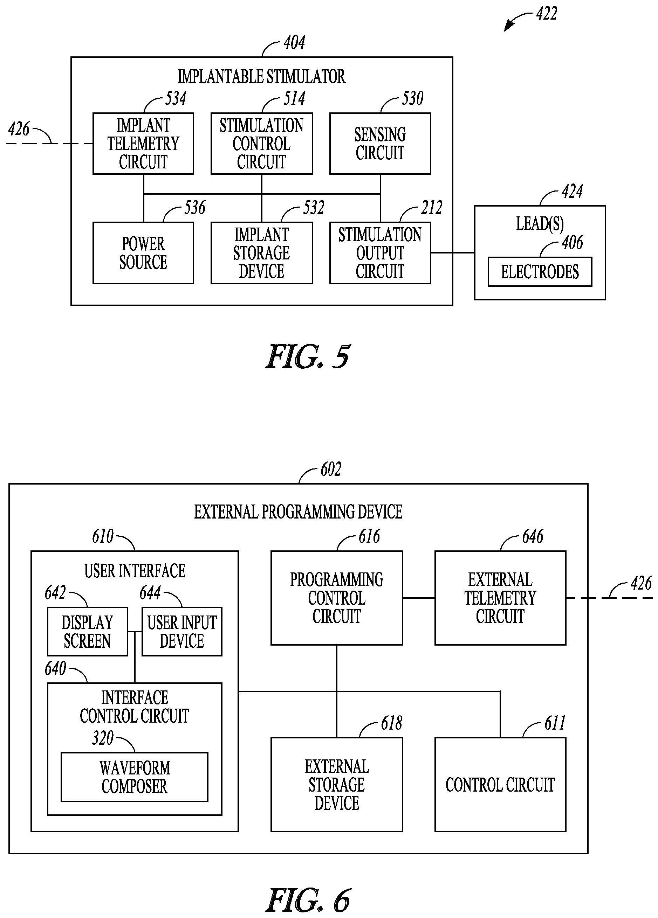

FIG. 5 illustrates, by way of example and not limitation, an example of the implantable stimulator 404 and one or more leads 424 of an implantable neurostimulation system, such as the implantable system 422. Implantable stimulator 404 may include a sensing circuit 530 that is optional and required only when the stimulator has a sensing capability, a stimulation output circuit 212, a stimulation control circuit 514, an implantable storage device 532, an implantable telemetry circuit 534, and a power source 536. The sensing circuit 530, when included and needed, may sense one or more physiological signals, such as for the purposes of patient monitoring and/or feedback control of the neurostimulation. Examples of the one or more physiological signals may include neural and other signals each indicative of a condition of the patient that is treated by the neurostimulation and/or a response of the patient to the delivery of the neurostimulation. The sensing circuit 530 may sense an impedance of at least one electrode delivering neurostimulation to the patient. The sensing circuit 530 may provide the sensed impedance to the control circuit 611, such as via the telemetry link 426. The stimulation output circuit 212 may be electrically connected to the electrodes 406 through the lead 424, and may deliver the neurostimulation through a set of electrodes selected from electrodes 406. The stimulation control circuit 514 may represent an embodiment of the stimulation control circuit 214 and may control the delivery of the neurostimulation using the plurality of stimulation parameters specifying a waveform shape or a pattern of neurostimulation pulses. In an example, the stimulation control circuit 514 may control the delivery of the neurostimulation using the one or more sensed physiological signals. The implant telemetry circuit 534 may provide the implantable stimulator 404 with wireless communication with another device such as a device of external system 402, including receiving values of the plurality of stimulation parameters from external system 402. The implant storage device 532 may store values of the plurality of stimulation parameters. The power source 536 may provide the implantable stimulator 404 with energy for its operation. In an example, the power source 536 includes a battery. In an example, the power source 536 includes a rechargeable battery and a battery charging circuit for charging the rechargeable battery. The implant telemetry circuit 534 may also function as a power receiver that receives power transmitted from external system 402 through an inductive couple.

In various examples, the sensing circuit 530 (if included), the stimulation output circuit 212, the stimulation control circuit 514, the implant telemetry circuit 534, the implant storage device 532, and the power source 536 are encapsulated in a hermetically sealed implantable housing. In various examples, the lead(s) 424 may be implanted such that the electrodes 406 are placed on and/or around one or more targets to which the neurostimulation is to be delivered, while implantable stimulator 404 is subcutaneously implanted and connected to lead(s) 424 at the time of implantation.

FIG. 6 illustrates an example of an external programming device 602 of an implantable neurostimulation system, such as the external system 402. The external programming device 602 may represent an embodiment of the programming device 302, and may include an external telemetry circuit 646, an external storage device 618, a programming control circuit 616, a control circuit 611, and a user interface 610.

The external telemetry circuit 646 may provide the external programming device 602 with wireless communication with another device such as the implantable stimulator 404 via telemetry link 426, including transmitting the plurality of stimulation parameters to the implantable stimulator 404. In one embodiment, the external telemetry circuit 646 also transmits power to the implantable stimulator 404 through the inductive couple.

The external storage device 618 may store a plurality of waveform building blocks each selectable for use as a portion of the pattern of the neurostimulation. In various embodiments, each waveform building block of the plurality of waveform building blocks includes one or more waveform shape of the neurostimulation, and may include one or more other waveform building blocks of the plurality of waveform building blocks. Examples of such waveforms include pulses, bursts each including a group of the pulses, trains each including a group of the bursts, and sequences each including a group of the pulses, bursts, and trains. The external storage device 618 may also store a plurality of stimulation fields. Each waveform building block of the plurality of waveform building blocks may associated with one or more fields of the plurality of stimulation fields. Each field of the plurality of stimulation fields may be defined by one or more electrodes of the plurality of electrodes through which the neurostimulation may be delivered and a current distribution of the pulse over the one or more electrodes.

The programming control circuit 616 represents an embodiment of the programming control circuit 316 and may generate the plurality of stimulation parameters, which may be transmitted to the implantable stimulator 404, according to the pattern of the neurostimulation. The pattern may be defined using one or more waveform building blocks selected from the plurality of waveform building blocks stored in the external storage device 618. In various embodiment, the programming control circuit 616 may check values of the plurality of stimulation parameters against safety rules to limit these values within constraints of the safety rules. In an example, the safety rules are heuristic rules.

The user interface 610 may represent an embodiment of the user interface 310 and may allow the user to define the pattern of neurostimulation pulses and perform various other monitoring and programming tasks. In an example, the user interface 610 includes a GUI. The user interface 610 includes a display screen 642, a user input device 644, and an interface control circuit 640. The display screen 642 may include any type of interactive or non-interactive screens, and the user input device 644 may include any type of user input devices that supports the various functions discussed in this document, such as touchscreen, keyboard, keypad, touchpad, trackball, joystick, and mouse. In an example, the user interface 610 may include a GUI that has an interactive screen for displaying a graphical representation of a waveform building block and may allow the user to adjust the waveform building block by graphically editing the waveform building block. The user interface 610 may also allow the user to perform any other functions discussed in this document where graphical editing is suitable as may be appreciated by those skilled in the art.

The interface control circuit 640 may control the operation of the user interface 610 including responding to various inputs received by the user input device 644 and defining the one or more stimulation waveforms. The interface control circuit 640 may include the waveform composer 320.

The external programming device 602 may have operation modes including a composition mode and a real-time programming mode. In the composition mode (also known as the pulse pattern composition mode), the user interface 610 may be activated, while the programming control circuit 616 may be deactivated. In an example, the programming control circuit 616 does not dynamically update values of the plurality of stimulation parameters in response to any change in the one or more stimulation waveforms. In the real-time programming mode, both the user interface 610 and the programming control circuit 616 may be activated. The programming control circuit 616 may dynamically update values of the plurality of stimulation parameters in response to changes in the set of one or more stimulation waveforms, and transmit the plurality of stimulation parameters with the updated values to the implantable stimulator 404. The control circuit 611 may receive a signal and may adjust the values of the plurality of stimulation parameters based on the received signal. The received signal may include information about a patient sensitivity to the stimulation waveform. The control circuit 611 may determine at least one stimulation parameter based on the information about the patient sensitivity to the stimulation waveform. The received signal may include a sensed impedance received from the sensing circuit 530. The control circuit may determine a relative electrode position based on the received sensed impedance received from the sensing circuit 530. The received signal may include an imaging signal received from an imaging device. The control circuit may determine a relative electrode position based on the received signal from the imaging device. The electrode may be an implanted electrode within the patient or may be external to the patient. The control circuit 611 may determine at least one stimulation parameter based on the determined relative position of the electrode.

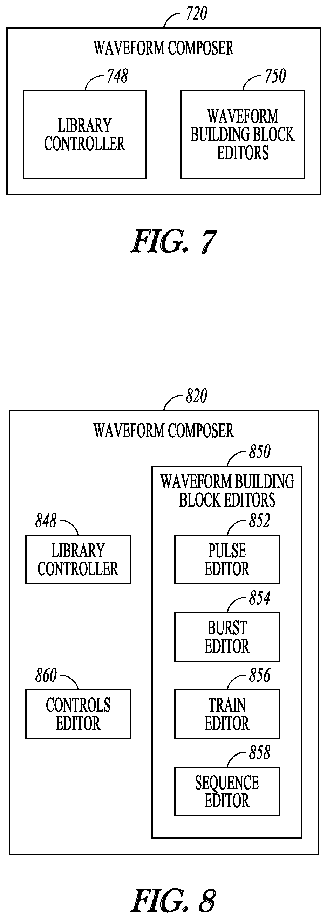

FIG. 7 illustrates an example of a waveform composer 720, which may represent an embodiment of the waveform composer 320. The waveform composer 720 may allow for composition of one of more waveform building blocks stored in the external storage device 618 and composition of the pattern of the neurostimulation using one of more waveform building blocks selected from the building blocks stored in external storage device 618. The waveform composer may include a library controller 748 and a plurality of waveform building block editors 750. The library controller 748 may display a library management area on the display screen 642. The displayed library management area may allow the user to manage the waveform building blocks stored in the external storage device 618. The waveform building block editors 750 may each display a composition area for a type of the waveform building blocks of a plurality of types of the waveform building blocks on display screen 642. The displayed composition area may allow the user to compose each waveform building block of the type of the waveform building blocks.

The waveform building block editors 750 may each display a composition area for a type of the waveform building blocks of the plurality of types of the waveform building blocks on the display screen 642 in response to a user selection of the type of the waveform building blocks. The displayed composition area allows the user to compose a waveform building block of the type of the waveform building blocks. The composition of the waveform building block may include editing a waveform building block selected from the waveform building blocks stored in the external storage device 618 or creating a new waveform building block to be added to the waveform building blocks stored in the external storage device 618.

Each type of the plurality of types of the waveform building blocks may represent a level of a plurality of levels of the waveform building blocks. A waveform building block of each level may include one or more waveform building blocks of one or more lower levels. In various embodiments, the plurality of types of the waveform building blocks may include, but is not limited to, pulses (e.g., each having a time scale of milliseconds), bursts (e.g., each having a time scale of milliseconds to seconds) each including a group of the pulses, trains (e.g., each having a time scale of milliseconds to minutes) each including a group of the bursts, and sequences (e.g., each having a time scale of minutes to weeks) each including a group of the pulses, bursts, and trains. Pauses (each being a time interval during which no pulse is to be delivered) may each be placed between two of the waveform building blocks. The sequences may each be composed for a specific therapy. The levels in the order from the lowest to the highest: pulse, burst, train, and sequence.

The waveform composer 720 may allow each of the library controller 748 and editors of the plurality of waveform building block editors 750 to be selected by the user for access. In one embodiment, the waveform composer 720 displays on the display screen 642 tags each associated with one of the library controller and the editors of the plurality of waveform building block editors, and allows the user to select a tag from the displayed tags. In an example, the waveform composer 720 may allow the delivery of the neurostimulation pulses to be turned on and off, such that the user can decide whether to suspend the delivery of the neurostimulation pulses during the composition of waveform building blocks and/or composition of the pattern of the neurostimulation pulses.

FIG. 8 illustrates an example of a waveform composer 820, which may represent an embodiment of the waveform composer 720. The waveform composer 820 may include a library controller 848, a plurality of waveform building block editors 850, and a controls editor 860.

The library controller 848 may represent an embodiment of the library controller 748 and may display a library management area on the display screen 642 in response to a user selection for access to the library controller.

The waveform building block editors 850 may represent an embodiment of the waveform building block editors 750, and include an editor for each type of the plurality of types of waveform building blocks. The waveform building block editors 850 may include a pulse editor 852, a burst editor 854, a train editor 856, and a sequence editor 858.

The pulse editor 852 may display a pulse composition area on display screen 642 in response to a user selection for access to the pulse editor. The pulse composition area may allow the user to compose a pulse of the waveform building blocks. The pulse composition area may allow the user to edit a pulse selected from the waveform building blocks stored in the external storage device 618 and to create a new pulse to be added to the waveform building blocks stored in the external storage device 618. The pulse composition area may display a graphical representation of the pulse being edited or created and a slider for shifting, expanding, or contracting a timeline of the graphical representation of the pulse. The pulse composition area may allow the user to select a pulse editing mode from a plurality of pulse editing modes, such as by displaying a pull down menu listing the plurality of pulse editing modes as illustrated. Examples of the pulse editing modes include, but are not limited to, a guided mode, a free form mode, and a draw mode. Under the guided mode, values of parameters defining the pulse are displayed, and the user is allowed to edit the pulse by adjusting the displayed values of the parameters. Under the free form mode, the user is allowed to edit the pulse by graphically modifying the displayed graphical representation of the pulse. Under the draw mode, the user is allowed to sketch a waveform for the pulse. In response to a selection of automatic charge balancing by the user, pulse editor 842 can automatically modify the pulse for charge balancing.

The burst editor 854 may display a burst composition area on display screen 642 in response to a user selection for access to the burst editor. The burst composition area may allow the user to compose a burst of the waveform building blocks. The burst composition area may allow the user to edit a burst selected from the waveform building blocks stored in the external storage device 618 or to create a new burst to be added to the waveform building blocks stored in the external storage device 618. The burst composition area may display a preview of a waveform of the burst and allows for saving of modified waveform of the burst. The burst composition area may allow the user to select options for editing each of the characteristics of the burst, such as duration, location (location in the body of the patient to which the burst is applied, i.e., electrode configuration), pulse frequency, pulse type, and pulse amplitude.

The train editor 846 may display a train composition area on the display screen 642 in response to a user selection for access to the train editor. The train composition area may allow the user to compose a train of the waveform building blocks. The train composition area may allow the user to edit a train selected from the waveform building blocks stored in the external storage device 618 or to create a new train to be added to the waveform building blocks stored in the external storage device 618. The train composition area may display a preview of a waveform of the train and may allow for saving of modified waveform of the train. The train composition area may allow the user to select options for editing each of the characteristics of the train, such as duration, burst location, burst frequency, train configuration, and burst amplitude.

The sequence editor 848 may display a sequence composition area on the display screen 642 in response to a user selection for access to the sequence editor. The sequence composition area may allow the user to compose a sequence of the waveform building blocks. The sequence composition area may allow the user to edit a sequence selected from the waveform building blocks stored in the external storage device 618 or to create a new sequence to be added to the waveform building blocks stored in the external storage device 618. The sequence composition area may display a preview of a waveform and may allow for saving of the modified waveform of the sequence. The sequence composition area may allow for selection of a sequence editing option from a plurality of sequence editing modes, and may allow for addition and deletion of waveform building blocks (sequence components) in the sequence, and may allow for simple editing of the waveform building blocks within the sequence composition area.

The controls editor 860 may display a controls area on the display screen 642 in response to a user command. The controls area allows the user to edit pulse parameters used for a waveform building block. The control area may allow the user to select a waveform building block and may apply various parameters to the selected waveform building block. The parameters may include a pulse amplitude, a pulse width, and a pulse frequency. The control area may allow the user to select advanced control including advanced editing options listed in a pull down menu, select coordinated reset for applying the edited pulse parameters (custom controls) to the selected waveform building block, and select how a parameter is defined for the selected waveform building block.

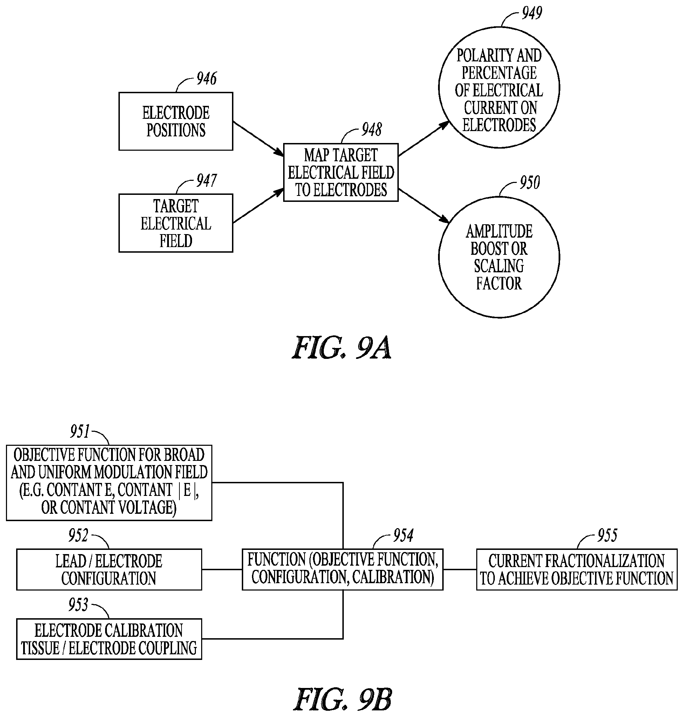

With reference to FIG. 9A, the external programming device 602 may be configured to accept relative electrode positions 946 and a representation of a target electrical field 947 and map the target electrical field to at least one electrode, such as the electrodes 406, thereby yielding the polarities and percentages of electrical current to be associated with the electrodes 949, as well as an amplitude boost or scaling factor 950 for globally adjusting the magnitude of the total current supplied to the electrodes to maintain a perceived intensity level of the electrical stimulation. Electrode locations and information about the desired electrical field may be independently input into the external programming device 602. The relative electrode positions may be received from the control circuit 311 or the control circuit 611.

FIG. 9B illustrates, by way of example, an embodiment for determining fractionalization to achieve an objective function. An objective function may refer to a function with desirable characteristics for modulating the targeted tissue. The objective function may also be referred to as an objective target function. An objective function 951 for a broad and uniform modulation field may be identified for a given volume of tissue. Examples of an objective function may include a constant E (electric field), a constant |E| (electric field magnitude), and a constant voltage. The lead and electrode configuration 952 may also be identified, as well as calibration for electrode tissue coupling 953. A function 954 may be performed that is dependent on the objective function, the lead and electrode configuration and the calibration. The result of the function is the fractionalization of modulation energy (e.g. current) 955 for each electrode to achieve the objective function.

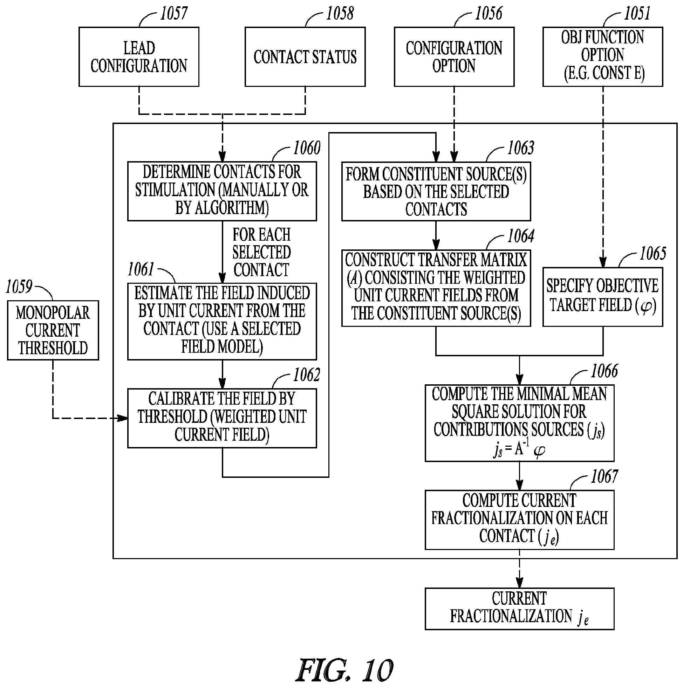

FIG. 10 illustrates, by way of example, an embodiment for determining fractionalization to achieve an objective function with more detail. An objective target function 1051 (e.g. constant E) is provided as an input to a process. Other inputs to the process include a configuration option 1056, a lead configuration 1057 and electrode contact status 1058, and a threshold 1059 such as a current threshold or more particularly a monopolar current threshold. The lead configuration 1057 and contact status 1058 identify an electrode arrangement, identifying a position of each electrode to determine the field. The overall field is a superimposed field from each electrode. The configuration option 1056 refers to monopolar (same polarity for all activated electrodes) and multipolar options (combined anode and cathodes in field). The threshold is used to compensate for electrode / tissue coupling differences.

The contacts for stimulation may be determined automatically or manually 1060 from the lead configuration and contact status. A selected field model may be used to estimate the field induced by unit current from the contact 1061. The field is calibrated using the threshold 1062. For example, the unit current field may be weighted. Constituent forces are formed based on the selected contacts 1063, and a transfer matrix 1064 is constructed to use to compute the minimal mean square solution 1066 using contributions from the constituent sources and using a specified target field 1065. The solution can be used to compute the current fractionalization on each contact 1067.

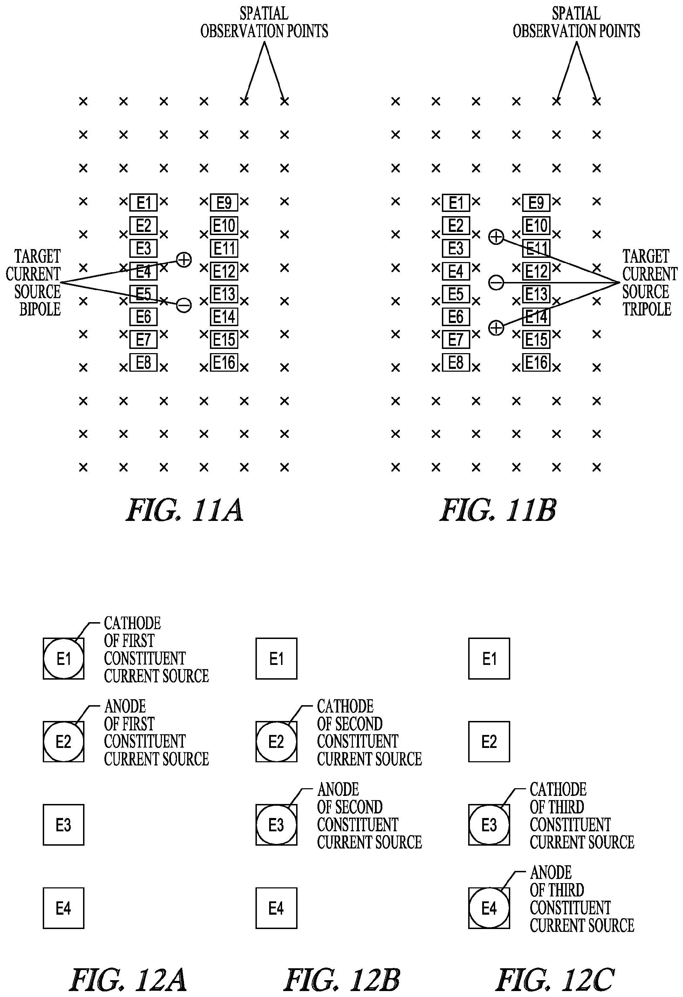

With reference to FIGS. 11A-11B, the external programming device 602 may map a target electrical field to the electrode array by estimating the field potential values (or some other linear electrical parameter, such as an activating function, current density, etc.) of the target field at a plurality of spatial observation points. The external programming device 602 may accomplish this by determining the desired locations of target current source poles relative to the electrode array 406, and modeling an electrical field generated by the target current source poles to determine desired field potential values at the spatial observation points (e.g., using analytical and/or numerical models).

Although target current source poles are one way to represent a "target electrical field", other representations of target fields may be used. The locations of the target current source poles may be determined in a manner that places the resulting electrical field over an identified region of the patient to be stimulated. The spatial observation points may be spaced in a manner that would, at the least, cover the entire tissue region to be stimulated and/or a tissue region that should not be stimulated. The locations of the target current source poles may be defined by the user, and may be displayed to the user along with the electrode locations, which as briefly discussed above, may be determined based on electrical measurements taken at the electrodes. Referring to FIGS. 12A -12C, the external programming device 602 may select, or allow a user to select, a plurality of constituent current sources at the locations of the electrodes. The locations of the electrodes may be determined based on measurements taken at the electrodes in response to sub-threshold electrical signals transmitted between the electrodes. In the illustrated target bipole a first constituent current source can be defined at the locations of electrodes E1 and E2 as -100% and + 100%, respectively (FIG. 12A); a second constituent current source can be defined at the locations of electrodes E2 and E3 as -100% and +100%, respectively (FIG. 12B); a third constituent current source can be defined at the locations of electrodes E3 and E4 as -100% and + 100%, respectively (FIG. 12C); and so on. The location of each of the electrodes is included within at least one of the constituent sources. Thus, the minimum number of constituent sources may be equal to the number of contacts less one, or may equal the number of contacts (e. g., if a monopole is used as the constituent source).

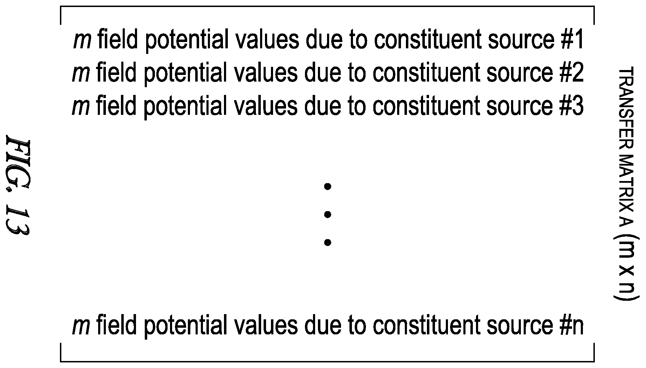

Once the constituent sources are selected, the external programming device 602 may determine the relative strengths of the constituent current sources that, when combined, result in estimated electrical field potential values at the spatial observation points that best match the desired field potential values at the spatial observation points. In particular, the external programming device 602 may model the constituent current sources (e.g., using analytical and/or numerical models) and estimate the field potential values per unit current (V/mA) generated by each of the constituent current sources at the spatial observation points, and may generate an m x n transfer matrix (shown in FIG. 13) from the estimated field potential values per unit current, with m equaling the number of spatial observation points and n equaling the number of constituent sources. The relative strengths of the constituent current sources may be determined using an optimization function that includes the transfer matrix A and the desired field potential values.

The optimization function may be a least-squares (over-determined) function expressed as: |φ-Aĵ|², where φ is an m-element vector of the desired field potential values, A is the transfer matrix, and j is an n-element vector of the strengths of the constituent current sources. The constituent current source strengths j may be solved such that the optimization function |φ-Aĵ|² is minimized. The square of the difference is minimized if φ=Aĵ. One approach for solving this problem may be to invert the transfer matrix A and pre-multiply, such that A⁻¹=φA⁻¹Aĵ, which yields the solution ĵ=A⁻¹φ. Once the strengths of the constituent current sources are determined, the external programming device 602 converts these strengths to current distributions on the electrodes in the form of a polarity and percentage.

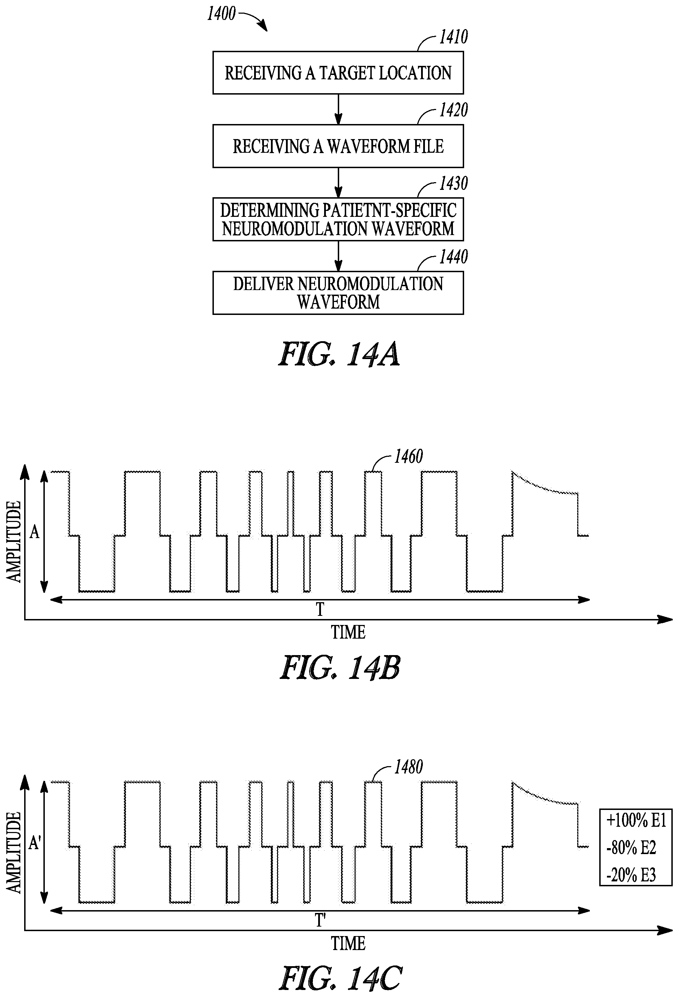

FIG. 14A illustrates, by way of example and not limitation, a method for adapting a generic neuromodulation waveform to a specific patient. The illustrated method may advantageously provide an improved method for adapting a generic neuromodulation waveform to a particular patient. The generic neuromodulation waveform may include a collection of pulses, bursts of pulses, trains of bursts, and sequences of pulses, bursts, and trains. The generic neuromodulation waveform may include a target neuromodulation field location (e.g., T7). The generic neuromodulation waveform may be composed by a user using a waveform composer, such as the waveform composer 320, 720, or 820. The generic neuromodulation waveform may be stored in a memory, such as the storage device 318 or the external storage device 618. A user may retrieve the stored generic neuromodulation waveform via a user interface, such as the user interface 110, 310 or 610 to provide the generic neuromodulation waveform to a specific patient. The retrieved generic neuromodulation waveform may be normalized (e.g., a value of a maximum amplitude of the generic neuromodulation waveform may be one, and the time duration of the neuromodulation waveform may be scaled temporally such that a total time duration of the waveform is equal to a predetermined time duration). A user interface, such as the user interface 110, 310 or 610 may receive a target neuromodulation field location from a user (step 1410). The user interface, such as the user interface 110, 310 or 610 may receive a waveform file that includes a generic neuromodulation waveform (step 1420). A control circuit, such as control circuit 311 or control circuit 611 may then determine a patient specific neuromodulation waveform (step 1430). The control circuit may determine the patient specific neuromodulation waveform based on patient feedback (e.g., paresthesia or tingling felt by the patient at a desired target location). The generic neuromodulation waveform can be delivered to a patient, and based on feedback received from the patient, the control circuit can determine at least one field parameter of the generic neuromodulation waveform. In an example, the control circuit can determine at least one waveform parameter based on patient feedback (e.g., a patient sensitivity threshold to the delivered neuromodulation). The control circuit may determine the patient specific neuromodulation waveform based on a determined electrode position within the patient. The control circuit may determine field parameters (e.g., current fractionalization or energy allocation) and waveform parameters (e.g., amplitude, frequency) based on the determined electrode position within the patient. The control circuit may adjust parameters of the generic neuromodulation waveform based on the determined field parameters and the determined waveform parameters to adapt the generic neuromodulation waveform to the patient. The shape and/or morphology of the generic neuromodulation waveform may be unchanged by adjustments made by the control circuit (e.g., defined relationships within the generic neuromodulation waveform are preserved) In an example, defined relationships may include a relationship between one or more amplitude values associated with stimulation pulses or bursts of stimulation pulses. In an example, defined relationships may include inter-stimulation pulse intervals. In an example, defined relationships may include the time ordering of stimulation pulses on a particular set of electrodes (e.g., a pulse pattern on electrodes 1-4, followed by a pulse pattern on electrodes 5-8). In an example, defined relationships may include a frequency or rate of application of stimulation waveforms. In an example, one or more waveform parameters may be stochastically or randomly modulated in response to adjustments made by the control circuit. In an example, the defined relationships may include any combination of the defined relationships described herein. The electrode position within the patient can be determined based on impedance measurements, such as can be performed by the implantable stimulator 404. In an example, the electrode position within the patient may be determined based on an imaging process. Electrodes, such as electrodes 106, 206, or 406 may then deliver the patient specific neuromodulation waveform to the patient (step 1440). In an example, the waveform parameters may be fixed and the control circuit may determine the field parameters based on feedback received from the patient. In an example, the field parameters may be fixed and the control circuit may determine the waveform parameters based on feedback received from the patient.

In an example, a generic neuromodulation waveform can provide deep brain stimulation to a patient with certain side effect(s) suppressed or reduced. The generic waveform can then be adapted to a specific patient based on a host of parameters. In an example, a generic neuromodulation waveform can provide peripheral nerve stimulation to a patient. In an example, a generic neuromodulation waveform can stimulate specific sensory and motor targets and patient. In an example, a generic neuromodulation waveform can provide vagal nerve stimulation. In an example, a generic neuromodulation waveform can provide a physiological effect to reduce hunger while keeping basal metabolic rate and blood pressure in normal ranges, and can be adapted to a specific patient based on individual anatomy, morphology and other patient to patient variations. In an example, a generic neuromodulation waveform can be applied in a motor control application where a lead may be placed in the spinal cord, motor cortex, or at the site of peripheral nerve (e.g., muscle). In an example, a generic neuromodulation waveform can provide complex stimulation waveform patterns or paradigms and may be utilized to perform or initiate peripheral limb motion or actions. In an example, a generic neuromodulation waveform can be customized by the patient.

FIG. 14B illustrates an example of a generic neuromodulation waveform 1460. The generic neuromodulation waveform 1460 may include a peak-to-peak amplitude of A, where the peak-to-peak amplitude A may be normalized, such as to a value of one. The generic neuromodulation waveform 1460 may include a time duration of T, where the time duration T may be normalized, such as to a predetermined time duration. After a control circuit, such as the control circuit 311 or 611 determines a patient sensitivity based on feedback received from the patient or an electrode position within the patient, the parameters of the generic neuromodulation waveform 1460 may be adjusted, such as to provide a patient specific neuromodulation waveform as shown in FIG. 14C. FIG. 14C illustrates an example of a patient-specific neuromodulation waveform 1480, where the generic neuromodulation waveform has been adjusted to provide the patient-specific neuromodulation waveform 1480. The patient specific neuromodulation waveform 1480 may include a peak-to-peak amplitude of A', where the peak-to-peak amplitude A' may be determined based on a patient sensitivity threshold. In an example, the amplitude A' may be determined based on the difference between a maximum patient sensitivity threshold and a minimum patient sensitivity threshold. The patient-specific neuromodulation waveform 1460 may include a time duration of T', where the time duration T' may be determined based on a patient specific dose of neuromodulation. A current fractionalization or energy allocation of the patient-specific neuromodulation waveform may be determined based on a received target field location and feedback received from the patient or a determined position of at least one implanted electrode within the patient. Delivering the neuromodulation waveform may include delivering 100% of an electrical current to a first electrode E1 and receiving a return electrical current of 80% from a second electrode E2, and 20% from a third electrode E3 to provide a neuromodulation field at the target field location.

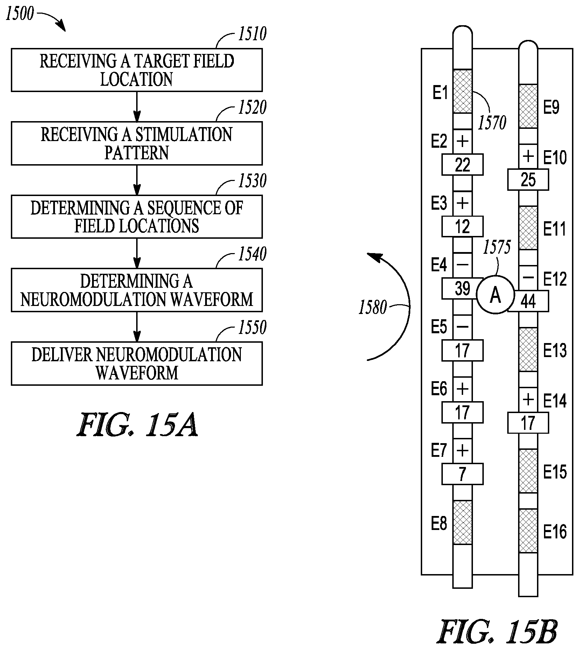

FIG. 15A illustrates, by way of example and not limitation, a method for providing a massage pattern to a patient. The illustrated method may advantageously provide a neuromodulation waveform to a patient, such as to provide a massage to the patient. The neuromodulation waveform may include a collection of pulses, bursts of pulses, trains of bursts, and sequences of pulses, bursts, and trains. The neuromodulation waveform may be composed by a user using a waveform composer, such as the waveform composer 320, 720, or 820. The neuromodulation waveform may be stored in a memory, such as the storage device 318 or the external storage device 618. A user may retrieve the stored neuromodulation waveform via a user interface, such as the user interface 110, 310 or 610 to provide the neuromodulation waveform to the patient. A user interface, such as the user interface 110, 310 or 610 may receive a first target neuromodulation field location and a stimulation pattern. (steps 1510 and 1520). A control circuit, such as control circuit 311 or control circuit 611 may then determine a sequence of target neuromodulation field locations based on the received first target neuromodulation field location and the stimulation pattern. (step 1530). The control circuit may determine the neuromodulation waveform based at least in part on a determined electrode position relative to the patient (step 1540). The control circuit may determine field parameters (e.g., current fractionalization or energy allocation) and waveform parameters (e.g., amplitude, frequency). Electrodes, such as electrodes 106, 206, or 406 may then deliver the determined neuromodulation waveform to provide the stimulation pattern to the patient (step 1550).

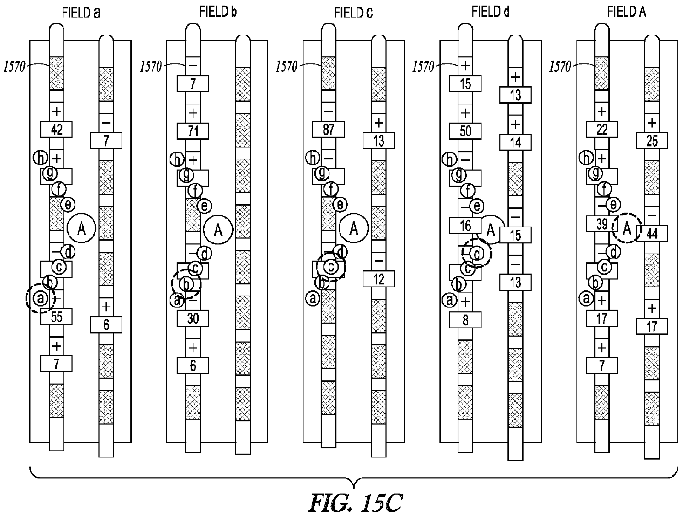

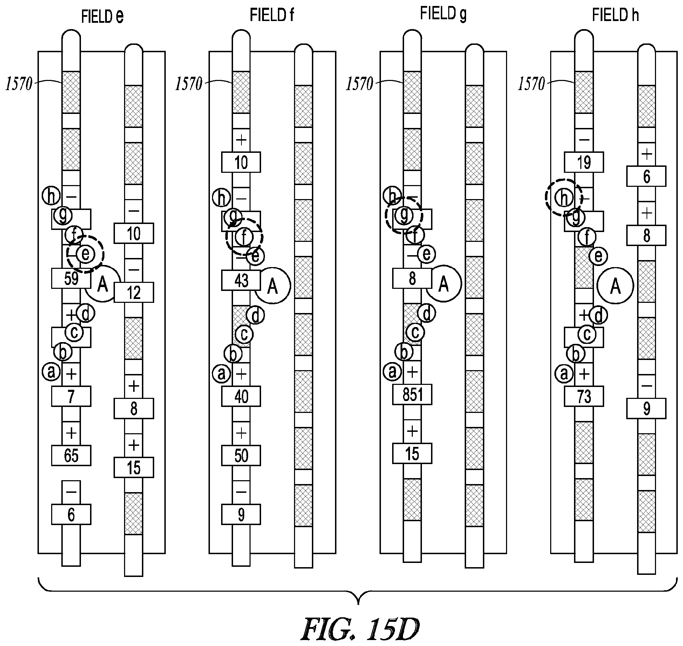

FIG. 15B illustrates by way of example, and not limitation an electrode configuration for providing neurostimulation to an initial target location. An initial target location ("A") 1575 may be received by a user interface, such the user interface 110, 310 or 610. A control circuit, such as control circuit 311 or control circuit 611 may determine a current value and polarity for each electrode in an array of electrodes 1570 (E1-E16), such as to provide neurostimulation at the initial target location. The user interface may also receive a stimulation pattern 1580. The stimulation pattern may include a spatial pattern. Based on the received initial target location and the received stimulation pattern, the control circuit may determine a collection of targets. The collection of targets may include the initial target location. For each target in the collection of targets, the control circuit may determine a current value and polarity for each electrode in the array of electrodes 1570. The control circuit may sequentially deliver a neurostimulation waveform to the collection of targets, such as to provide a time varying neurostimulation pattern to the patient. The time varying neurostimulation pattern may include a massage pattern. FIGS. 15C and 15D illustrate an example of a time varying neurostimulation pattern, according to the stimulation pattern and the target location illustrated in FIG. 15B. At a first time, the control circuit can configure the electrode array 1570, such as to deliver neurostimulation to a first target ("a") in the collection of targets. At a second time, the control circuit can configure the electrode array 1570, such as to deliver neurostimulation to a second target ("b") in the collection of targets. At a third time, the control circuit can configure the electrode array 1570, such as to deliver neurostimulation to a third target ("c") in the collection of targets. At a fourth time, the control circuit can configure the electrode array 1570, such as to deliver neurostimulation to a fourth target ("d") in the collection of targets. At a fifth time, the control circuit can configure the electrode array 1570, such as to deliver neurostimulation to a fifth target ("A") in the collection of targets. At a sixth time, the control circuit can configure the electrode array 1570, such as to deliver neurostimulation to a sixth target ("e") in the collection of targets. At a seventh time, the control circuit can configure the electrode array 1570, such as to deliver neurostimulation to a seventh target ("f") in the collection of targets. At an eighth time, the control circuit can configure the electrode array 1570, such as to deliver neurostimulation to an eighth target ("g") in the collection of targets. At a ninth time, the control circuit can configure the electrode array 1570, such as to deliver neurostimulation to a ninth target ("h") in the collection of targets. The control sequence may then repeat, such as to provide a time varying neurostimulation pattern to the patient in accordance with the received target location and stimulation pattern.

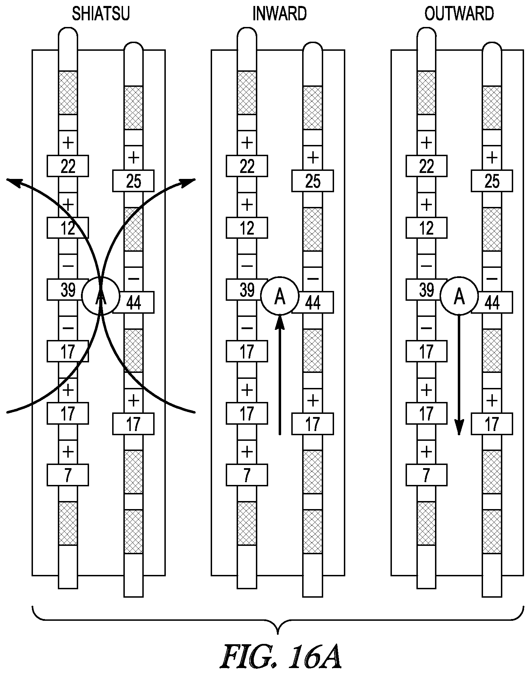

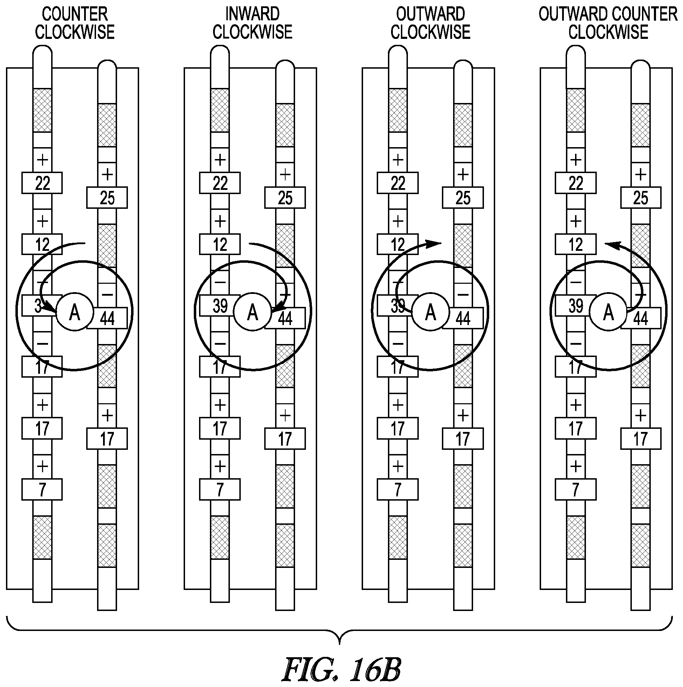

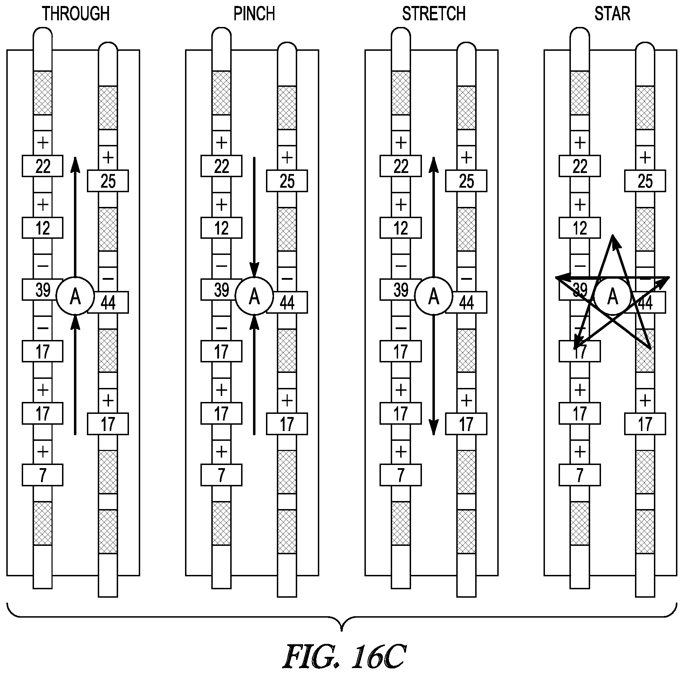

FIG. 16A illustrates examples of stimulation patterns, such as those provided by the array of electrodes 1570. The stimulation patterns may include a shiatsu pattern, an inward pattern, or an outward pattern. FIG. 16B illustrates examples of stimulation patterns, such as those provided by the array of electrodes 1570. The stimulation patterns may include a counterclockwise pattern, an inward clockwise pattern, an outward clockwise pattern, or an outward counter clockwise pattern. FIG. 16C illustrates example of stimulation patterns, such as those provided by the array of electrodes 1570. The stimulation patterns may include a trough pattern, a pinch pattern, a stretch pattern, or a star pattern. In an example where the stimulation pattern may include a trough pattern, an amplitude of a neurostimulation waveform delivered to the electrode array 1570 may increase as a distance from the target to the received initial target decreases. In an example where the stimulation pattern may include a pinch pattern, the control circuit may sequentially deliver a neurostimulation waveform to at least two targets at a time and an amplitude of a neurostimulation waveform delivered to the electrode array 1570 may increase as a distance from the targets to the received initial target decreases. In an example where the stimulation pattern may include a stretch pattern, the control circuit may sequentially deliver a neurostimulation waveform to at least two targets at a time, and the neurostimulation waveform may be delivered to targets that are

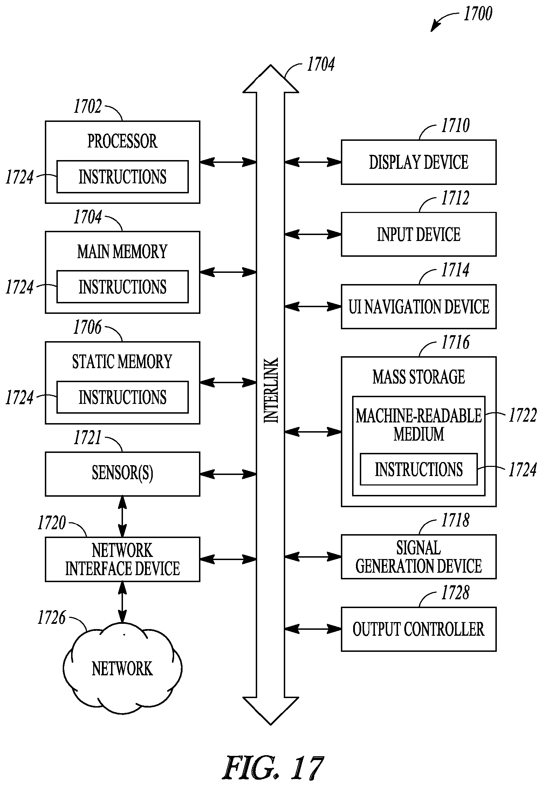

FIG. 17 illustrates generally a block diagram of an example machine 1700 upon which any one or more of the techniques (e.g., methodologies) discussed herein may perform. Portions of this description may apply to the computing framework of various portions of the LCP device, the IMD, or the external programmer.

In alternative embodiments, the machine 1700 may operate as a standalone device or may be connected (e.g., networked) to other machines. In a networked deployment, the machine 1700 may operate in the capacity of a server machine, a client machine, or both in server-client network environments. In an example, the machine 1700 may act as a peer machine in peer-to-peer (P2P) (or other distributed) network environment. The machine 1700 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 1700 may include a hardware processor 1702 (e.g., a central processing unit (CPU), a graphics processing unit (GPU), a hardware processor core, or any combination thereof), a main memory 1704 and a static memory 1706, some or all of which may communicate with each other via an interlink (e.g., bus) 1708. The machine 1700 may further include a display unit 1710 (e.g., a raster display, vector display, holographic display, etc.), an alphanumeric input device 1712 (e.g., a keyboard), and a user interface (UI) navigation device 1714 (e.g., a mouse). In an example, the display unit 1710, input device 1712 and UI navigation device 1714 may be a touch screen display. The machine 1700 may additionally include a storage device (e.g., drive unit) 1716, a signal generation device 1718 (e.g., a speaker), a network interface device 1720, and one or more sensors 1721, such as a global positioning system (GPS) sensor, compass, accelerometer, or other sensor. The machine 1700 may include an output controller 1728, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 1716 may include a machine readable medium 1722 on which is stored one or more sets of data structures or instructions 1724 (e.g., software) embodying or utilized by any one or more of the techniques or functions described herein. The instructions 1724 may also reside, completely or at least partially, within the main memory 1704, within static memory 1706, or within the hardware processor 1702 during execution thereof by the machine 1700. In an example, one or any combination of the hardware processor 1702, the main memory 1704, the static memory 1706, or the storage device 1716 may constitute machine readable media.

While the machine readable medium 1722 is illustrated as a single medium, the term "machine readable medium" may include a single medium or multiple media (e.g., a centralized or distributed database, and/or associated caches and servers) configured to store the one or more instructions 1724.

The term "machine readable medium" may include any medium that is capable of storing, encoding, or carrying instructions for execution by the machine 1700 and that cause the machine 1700 to perform any one or more of the techniques of the present disclosure, or that is capable of storing, encoding or carrying data structures used by or associated with such instructions. Nonlimiting machine readable medium examples may include solid-state memories, and optical and magnetic media. In an example, a massed machine readable medium comprises a machine readable medium with a plurality of particles having invariant (e.g., rest) mass. Accordingly, massed machine-readable media are not transitory propagating signals. Specific examples of massed machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 1724 may further be transmitted or received over a communications network 1726 using a transmission medium via the network interface device 1720 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 1720 may include one or more physical jacks (e.g., Ethernet, coaxial, or phone jacks) or one or more antennas to connect to the communications network 1726. In an example, the network interface device 1720 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1700, and includes digital or analog communications signals or other intangible medium to facilitate communication of such software.

Various embodiments are illustrated in the figures above. One or more features from one or more of these embodiments may be combined to form other embodiments.

Method examples described herein can be machine or computer-implemented at least in part. Some examples may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times.

## Claims

1. A system, comprising:
user interface circuitry configured to receive user input corresponding to a target neuromodulation field location within a patient; and
control circuitry configured to receive a waveform file which includes a generic neuromodulation waveform, wherein the generic neuromodulation waveform is a normalized neuromodulation waveform normalized to a defined amplitude value and time duration, and use the waveform file and the target neuromodulation field location to determine a patient-specific neuromodulation waveform to be delivered to at least one electrode to provide a neuromodulation field at the target neurostimulation field location.

2. The system according to claim 1, further comprising neurostimulation circuitry configured to deliver the patient-specific neuromodulation waveform to the at least one electrode to provide the neuromodulation field at the target neurostimulation field location.

3. The system according to any of claims 1-2, wherein the control circuitry is further configured to determine a position of at least one implanted electrode within the patient and determine at least one field modulation parameter value to adapt the waveform in the waveform file to a patient-specific neuromodulation waveform based on the determined position of the at least one implanted electrode.

4. The system according to any of claims 1-3 wherein the control circuitry is further configured to receive a feedback signal from the patient to determine a portion of the patient's body affected by the delivered neuromodulation waveform and determine at least one field modulation parameter value to adapt the waveform in the waveform file to a patient-specific neuromodulation waveform based on the determined portion of the patient's body affected by the delivered neuromodulation.

5. The system according to any of claims 1-4, further comprising sensor circuitry configured to determine a physiologic parameter of the patient and wherein the control circuitry is further configured to determine a position of at least one implanted electrode within the patient and determine at least one defined neuromodulation parameter value and at least one field modulation parameter value to adapt the waveform to a patient-specific neuromodulation waveform based on the determined physiologic parameter of the patient and the determined position of the at least one implanted electrode within the patient.

6. The system according to any of claims 1-5, further comprising an external device, wherein the external device includes the user interface circuitry and the control circuitry, and further includes communication circuitry operably connected to the control circuitry to communicate data indicative of the patient-specific neuromodulation waveform to a neurostimulator device.

7. The system according to any of claims 1-6, further comprising an external device and a neurostimulator device, wherein the external device includes the user interface circuitry, and the external device is configured to communicate data indicative of the target neuromodulation field location and the waveform file to the neurostimulator device, and the neurostimulator device includes the control circuitry to use the waveform file and the target neuromodulation field location to determine the patient-specific waveform, the neurostimulator device configured to deliver the patient-specific waveform to the at least one electrode.

8. A non-transitory machine-readable medium including instructions, which when executed by a machine, cause the machine to:
receive user input corresponding to a target neuromodulation field location within a patient for a neuromodulation field;
receive a waveform file which includes a generic neuromodulation waveform, wherein the generic neuromodulation waveform is a normalized neuromodulation waveform normalized to a defined amplitude value and time duration;
use the waveform file and the target neuromodulation field location to determine a patient-specific neuromodulation waveform; and
generate a signal corresponding to the patient-specific neuromodulation waveform for the at least one electrode to provide a neuromodulation field at the target neurostimulation field location.

9. The non-transitory machine-readable medium according to claim 8, wherein using the waveform file and the target neuromodulation field location to determine a patient-specific neuromodulation waveform comprises:
determining a position of at least one implanted electrode within the patient; and
determining at least one field modulation parameter value to adapt the waveform in the waveform file to a patient-specific neuromodulation waveform based on the determined position of the at least one implanted electrode.

10. The non-transitory machine-readable medium according to any of claims 8-9, wherein using the waveform file and the target neuromodulation field location to determine a patient-specific neuromodulation waveform comprises:
delivering a neuromodulation waveform to the patient based on the received waveform file and the target neuromodulation field;
receiving a feedback signal from the patient to determine a portion of the patient's body affected by the delivered neuromodulation waveform; and
determining at least one field modulation parameter value to adapt the waveform in the waveform file to a patient-specific neuromodulation waveform based on the determined portion of the patient's body affected by the delivered neuromodulation.

11. The non-transitory machine-readable medium according to any of claims 8-10, further comprising instructions, which when executed by a machine, cause the machine to move the target neuromodulation field location, and adjust at least one field modulation parameter value for the neuromodulation field to maintain defined relationships in the waveform when the target neuromodulation field location is moved

12. The non-transitory machine-readable medium according to any of claims 8-11, wherein the waveform includes defined neuromodulation parameters, the method further comprising adjusting at least one defined neuromodulation parameter of the waveform and adjusting at least one field modulation parameter value for the neuromodulation field to maintain defined relationships in the waveform when the at least one defined neuromodulation parameter of the waveform is adjusted.

13. The non-transitory machine-readable medium according to any of claims 8-12, wherein the neuromodulation waveform comprises a plurality of waveform segments and using the waveform file and the target neuromodulation field location to determine a patient-specific neuromodulation waveform includes determining at least one field modulation parameter value for each waveform segment.

14. The non-transitory machine-readable medium according to any of claims 8-13, wherein using the waveform file and the target neuromodulation field location to determine a patient-specific neuromodulation waveform includes:
determining a patient sensitivity to neuromodulation at the target neurostimulation field location;
using the determined patient sensitivity to determine a target neuromodulation dose range;
selecting at least one defined neuromodulation parameter value to provide a patient-specific dose for the patient-specific neuromodulation waveform that is within the target neuromodulation dose range.

15. The non-transitory machine-readable medium according to any of claims 8-14, further comprising instructions, which when executed by a machine, cause the machine to adjust at least one field modulation parameter value of the waveform and adjust the at least one defined neuromodulation parameter value to maintain defined relationships in the waveform when the at least one field modulation parameter of the waveform is adjusted.

## Patentansprüche

1. System, aufweisend:
eine Benutzerschnittstellenschaltung, die dazu eingerichtet ist, eine Benutzereingabe zu empfangen, die einer Ziel-Neuromodulationsfeldposition innerhalb eines Patienten entspricht; und
eine Steuerschaltung, die dazu eingerichtet ist, eine Wellenformdatei zu empfangen, die eine generische Neuromodulationswellenform aufweist, wobei die generische Neuromodulationswellenform eine normalisierte Neuromodulationswellenform ist, die auf einen definierten Amplitudenwert und eine definierte Zeitdauer normalisiert ist, und die Wellenformdatei und die Ziel-Neuromodulationsfeldposition zu verwenden, um eine patientenspezifische Neuromodulationswellenform zu bestimmen, die mindestens einer Elektrode zugeführt werden soll, um ein Neuromodulationsfeld an der Ziel-Neurostimulationsfeldposition zu erzeugen.

2. System nach Anspruch 1, ferner aufweisend eine Neurostimulationsschaltung, die dazu eingerichtet ist, die patientenspezifische Neuromodulationswellenform der mindestens einen Elektrode zuzuführen, um das Neuromodulationsfeld an der Ziel-Neurostimulationsfeldposition bereitzustellen.

3. System nach Anspruch 1 oder 2, wobei die Steuerschaltung ferner dazu eingerichtet ist, eine Position mindestens einer implantierten Elektrode innerhalb des Patienten zu bestimmen und mindestens einen Feldmodulationsparameterwert zu bestimmen, um die Wellenform in der Wellenformdatei basierend auf der bestimmten Position der mindestens einen implantierten Elektrode an eine patientenspezifische Neuromodulationswellenform anzupassen.

4. System nach einem der Ansprüche 1 bis 3, wobei die Steuerschaltung ferner dazu eingerichtet ist, ein Rückmeldungssignal vom Patienten zu empfangen, um einen durch die zugeführte Neuromodulationswellenform beeinflussten Körperbereich des Patienten zu bestimmen und mindestens einen Feldmodulationsparameterwert zu bestimmen, um die Wellenform in der Wellenformdatei basierend auf dem bestimmten, durch die zugeführte Neuromodulation beeinflussten Körperbereich des Patienten an eine patientenspezifische Neuromodulationswellenform anzupassen.

5. System nach einem der Ansprüche 1 bis 4, ferner aufweisend eine Sensorschaltung, die dazu eingerichtet ist, einen physiologischen Parameter des Patienten zu bestimmen, wobei die Steuerschaltung ferner dazu eingerichtet ist, eine Position mindestens einer implantierten Elektrode innerhalb des Patienten zu bestimmen und mindestens einen definierten Neuromodulationsparameterwert sowie mindestens einen Feldmodulationsparameterwert zu bestimmen, um die Wellenform basierend auf dem bestimmten physiologischen Parameter des Patienten und der bestimmten Position der mindestens einen implantierten Elektrode innerhalb des Patienten an eine patientenspezifische Neuromodulationswellenform anzupassen.

6. System nach einem der Ansprüche 1 bis 5, ferner aufweisend eine externe Vorrichtung, wobei die externe Vorrichtung die Benutzerschnittstellenschaltung und die Steuerschaltung aufweist und ferner eine betrieblich mit der Steuerschaltung verbundene Kommunikationsschaltung zum Übertragen von Daten, die die patientenspezifische Neuromodulationswellenform anzeigen, an eine Neurostimulatorvorrichtung aufweist.

7. System nach einem der Ansprüche 1 bis 6, ferner aufweisend eine externe Vorrichtung und eine Neurostimulatorvorrichtung, wobei die externe Vorrichtung die Benutzerschnittstellenschaltung aufweist, und wobei die externe Vorrichtung dazu eingerichtet ist, Daten, die die Ziel-Neuromodulationsfeldposition und die Wellenformdatei anzeigen, an die Neurostimulatorvorrichtung zu übertragen, und wobei die Neurostimulatorvorrichtung die Steuerschaltung aufweist, um die Wellenformdatei und die Ziel-Neuromodulationsfeldposition zum Bestimmen der patientenspezifischen Wellenform zu verwenden, wobei die Neurostimulatorvorrichtung dazu eingerichtet ist, die patientenspezifische Wellenform der mindestens einen Elektrode zuzuführen.

8. Nichtflüchtiges, maschinenlesbares Medium, das Anweisungen aufweist, die, wenn sie durch eine Maschine ausgeführt werden, die Maschine veranlassen:
eine Benutzereingabe zu empfangen, die einer Ziel-Neuromodulationsfeldposition innerhalb eines Patienten für ein Neuromodulationsfeld entspricht;
eine Wellenformdatei zu empfangen, die eine generische Neuromodulationswellenform aufweist, wobei die generische Neuromodulationswellenform eine normalisierte Neuromodulationswellenform ist, die auf einen definierten Amplitudenwert und eine definierte Zeitdauer normalisiert ist;
die Wellenformdatei und die Ziel-Neuromodulationsfeldposition zum Bestimmen einer patientenspezifischen Neuromodulationswellenform zu verwenden; und
ein der patientenspezifischen Neuromodulationswellenform entsprechendes Signal für die mindestens eine Elektrode zu erzeugen, um ein Neuromodulationsfeld an der Ziel-Neurostimulationsfeldposition bereitzustellen.

9. Nichtflüchtiges, maschinenlesbares Medium nach Anspruch 8, wobei das Verwenden der Wellenformdatei und der Ziel-Neuromodulationsfeldposition zum Bestimmen einer patientenspezifischen Neuromodulationswellenform aufweist:
Bestimmen einer Position mindestens einer implantierten Elektrode innerhalb des Patienten; und
Bestimmen mindestens eines Feldmodulationsparameterwerts zum Anpassen der Wellenform in der Wellenformdatei basierend auf der bestimmten Position der mindestens einen implantierten Elektrode an eine patientenspezifische Neuromodulationswellenform.

10. Nichtflüchtiges, maschinenlesbares Medium nach Anspruch 8 oder 9, wobei das Verwenden der Wellenformdatei und der Ziel-Neuromodulationsfeldposition zum Bestimmen einer patientenspezifischen Neuromodulationswellenform aufweist:
Zuführen einer Neuromodulationswellenform zum Patienten basierend auf der empfangenen Wellenformdatei und der Ziel-Neuromodulationsfeldposition;
Empfangen eines Rückmeldungssignals vom Patienten zum Bestimmen eines durch die zugeführte Neuromodulationswellenform beeinflussten Körperbereichs des Patienten; und
Bestimmen mindestens eines Feldmodulationsparameterwerts zum Anpassen der Wellenform in der Wellenformdatei basierend auf dem bestimmten, durch die zugeführte Neuromodulation beeinflussten Körperbereich des Patienten an eine patientenspezifische Neuromodulationswellenform.

11. Nichtflüchtiges, maschinenlesbares Medium nach einem der Ansprüche 8 bis 10, das ferner Anweisungen aufweist, die, wenn sie durch eine Maschine ausgeführt werden, die Maschine veranlassen, die Ziel-Neuromodulationsfeldposition zu verschieben und mindestens einen Feldmodulationsparameterwert für das Neuromodulationsfeld anzupassen, um definierte Beziehungen in der Wellenform aufrechtzuerhalten, wenn die Ziel-Neuromodulationsfeldposition verschoben ist.

12. Nichtflüchtiges, maschinenlesbares Medium nach einem der Ansprüche 8 bis 11, wobei die Wellenform definierte Neuromodulationsparameter aufweist, wobei das Verfahren ferner das Anpassen mindestens eines definierten Neuromodulationsparameters der Wellenform und das Anpassen mindestens eines Feldmodulationsparameterwerts für das Neuromodulationsfeld aufweist, um definierte Beziehungen in der Wellenform aufrechtzuerhalten, wenn der mindestens eine definierte Neuromodulationsparameter der Wellenform angepasst ist.

13. Nichtflüchtiges, maschinenlesbares Medium nach einem der Ansprüche 8 bis 12, wobei die Neuromodulationswellenform eine Vielzahl von Wellenformsegmenten aufweist und das Verwenden der Wellenformdatei und der Ziel-Neuromodulationsfeldposition zum Bestimmen einer patientenspezifischen Neuromodulationswellenform das Bestimmen mindestens eines Feldmodulationsparameterwerts für jedes Wellenformsegment aufweist.

14. Nichtflüchtiges, maschinenlesbares Medium nach einem der Ansprüche 8 bis 13, wobei das Verwenden der Wellenformdatei und der Ziel-Neuromodulationsfeldposition zum Bestimmen einer patientenspezifischen Neuromodulationswellenform aufweist:
Bestimmen einer Patientensensitivität bezüglich der Neuromodulation an der Ziel-Neurostimulationsfeldposition;
Verwenden der bestimmten Patientensensitivität zum Bestimmen eines Ziel-Neuromodulationsdosisbereichs;
Auswählen mindestens eines definierten Neuromodulationsparameterwerts, zum Bereitstellen einer patientenspezifischen Dosis für die patientenspezifische Neuromodulationswellenform, die innerhalb des Ziel-Neuromodulationsdosisbereichs liegt.

15. Nichtflüchtiges, maschinenlesbares Medium nach einem der Ansprüche 8 bis 14, das ferner Anweisungen aufweist, die, wenn sie durch eine Maschine ausgeführt werden, die Maschine veranlassen, mindestens einen Feldmodulationsparameterwert der Wellenform anzupassen und den mindestens einen definierten Neuromodulationsparameterwert anzupassen, um definierte Beziehungen in der Wellenform aufrechtzuerhalten, wenn der mindestens eine Feldmodulationsparameter der Wellenform angepasst ist.

## Revendications

1. Système, comportant :
des circuits d'interface utilisateur configurés pour recevoir une entrée utilisateur correspondant à un emplacement cible de champ de neuromodulation chez un patient ; et
des circuits de commande configurés pour recevoir un fichier de forme d'onde qui comprend une forme d'onde de neuromodulation générique, la forme d'onde de neuromodulation générique étant une forme d'onde de neuromodulation normalisée à une valeur d'amplitude définie et à une durée temporelle définie, et utiliser le fichier de forme d'onde et l'emplacement cible de champ de neuromodulation pour déterminer une forme d'onde de neuromodulation spécifique au patient à délivrer à au moins une électrode afin de fournir un champ de neuromodulation à l'emplacement cible de champ de neurostimulation.

2. Système selon la revendication 1, comportant en outre des circuits de neurostimulation configurés pour délivrer la forme d'onde de neuromodulation spécifique au patient à l'au moins une électrode afin de fournir le champ de neuromodulation à l'emplacement cible de champ de neurostimulation.

3. Système selon l'une quelconque des revendications 1 à 2, dans lequel les circuits de commande sont en outre configurés pour déterminer une position d'au moins une électrode implantée chez le patient et déterminer au moins une valeur de paramètre de modulation de champ pour adapter la forme d'onde dans le fichier de forme d'onde en une forme d'onde de neuromodulation spécifique au patient sur la base de la position déterminée de l'au moins une électrode implantée.

4. Système selon l'une quelconque des revendications 1 à 3, dans lequel les circuits de commande sont en outre configurés pour recevoir un signal de rétroaction du patient afin de déterminer une partie du corps du patient affectée par la forme d'onde de neuromodulation délivrée et déterminer au moins une valeur de paramètre de modulation de champ pour adapter la forme d'onde dans le fichier de forme d'onde en une forme d'onde de neuromodulation spécifique au patient sur la base de la partie déterminée du corps du patient affectée par la neuromodulation délivrée.

5. Système selon l'une quelconque des revendications 1 à 4, comportant en outre des circuits de capteur configurés pour déterminer un paramètre physiologique du patient et dans lequel les circuits de commande sont en outre configurés pour déterminer la position d'au moins une électrode implantée chez le patient et déterminer au moins une valeur de paramètre de neuromodulation définie et au moins une valeur de paramètre de modulation de champ pour adapter la forme d'onde en une forme d'onde de neuromodulation spécifique au patient sur la base du paramètre physiologique déterminé du patient et de la position déterminée de l'au moins une électrode implantée chez le patient.

6. Système selon l'une quelconque des revendications 1 à 5, comportant en outre un dispositif externe, le dispositif externe comprenant les circuits d'interface utilisateur et les circuits de commande, et comprenant en outre des circuits de communication connectés de manière fonctionnelle aux circuits de commande pour communiquer des données indicatives de la forme d'onde de neuromodulation spécifique au patient à un dispositif neurostimulateur.

7. Système selon l'une quelconque des revendications 1 à 6, comportant en outre un dispositif externe et un dispositif neurostimulateur, le dispositif externe comprenant les circuits d'interface utilisateur, et le dispositif externe étant configuré pour communiquer des données indicatives de l'emplacement cible de champ de neuromodulation et du fichier de forme d'onde au dispositif neurostimulateur, et le dispositif neurostimulateur comprenant les circuits de commande pour utiliser le fichier de forme d'onde et l'emplacement cible de champ de neuromodulation afin de déterminer la forme d'onde spécifique au patient, le dispositif neurostimulateur étant configuré pour délivrer la forme d'onde spécifique au patient à l'au moins une électrode.

8. Support non transitoire lisible par machine comprenant des instructions qui, lorsqu'elles sont exécutées par une machine, amènent la machine à :
recevoir une entrée utilisateur correspondant à un emplacement cible de champ de neuromodulation chez un patient pour un champ de neuromodulation ;
recevoir un fichier de forme d'onde qui comprend une forme d'onde de neuromodulation générique, la forme d'onde de neuromodulation générique étant une forme d'onde de neuromodulation normalisée à une valeur d'amplitude définie et à une durée temporelle définie ;
utiliser le fichier de forme d'onde et l'emplacement cible de champ de neuromodulation pour déterminer une forme d'onde de neuromodulation spécifique au patient ; et
générer un signal correspondant à la forme d'onde de neuromodulation spécifique au patient pour l'au moins une électrode afin de fournir un champ de neuromodulation à l'emplacement cible de champ de neurostimulation.

9. Support non transitoire lisible par machine selon la revendication 8, dans lequel l'utilisation du fichier de forme d'onde et de l'emplacement cible de champ de neuromodulation pour déterminer une forme d'onde de neuromodulation spécifique au patient comprend :
la détermination d'une position d'au moins une électrode implantée chez le patient ; et
la détermination d'au moins une valeur de paramètre de modulation de champ pour adapter la forme d'onde dans le fichier de forme d'onde en une forme d'onde de neuromodulation spécifique au patient sur la base de la position déterminée de l'au moins une électrode implantée.

10. Support non transitoire lisible par machine selon l'une quelconque des revendications 8 à 9, dans lequel l'utilisation du fichier de forme d'onde et de l'emplacement cible de champ de neuromodulation pour déterminer une forme d'onde de neuromodulation spécifique au patient comprend :
la délivrance d'une forme d'onde de neuromodulation au patient sur la base du fichier de forme d'onde reçu et de l'emplacement cible de champ de neuromodulation ;
la réception d'un signal de rétroaction du patient afin de déterminer une partie du corps du patient affectée par la forme d'onde de neuromodulation délivrée ; et
la détermination d'au moins une valeur de paramètre de modulation de champ pour adapter la forme d'onde dans le fichier de forme d'onde en une forme d'onde de neuromodulation spécifique au patient sur la base de la partie déterminée du corps du patient affectée par la neuromodulation délivrée.

11. Support non transitoire lisible par machine selon l'une quelconque des revendications 8 à 10, comprenant en outre des instructions qui, lorsqu'elles sont exécutées par une machine, amènent la machine à déplacer l'emplacement cible du champ de neuromodulation, et à ajuster au moins une valeur de paramètre de modulation de champ pour le champ de neuromodulation afin de maintenir des relations définies dans la forme d'onde lorsque l'emplacement cible du champ de neuromodulation est déplacé.

12. Support non transitoire lisible par machine selon l'une quelconque des revendications 8 à 11, dans lequel la forme d'onde comprend des paramètres de neuromodulation définis, le procédé comprenant en outre l'ajustement d'au moins un paramètre de neuromodulation défini de la forme d'onde et l'ajustement d'au moins une valeur de paramètre de modulation de champ pour le champ de neuromodulation afin de maintenir des relations définies dans la forme d'onde lorsque ledit au moins un paramètre de neuromodulation défini de la forme d'onde est ajusté.

13. Support non transitoire lisible par machine selon l'une quelconque des revendications 8 à 12, dans lequel la forme d'onde de neuromodulation comprend une pluralité de segments de forme d'onde, et l'utilisation du fichier de forme d'onde et de l'emplacement cible du champ de neuromodulation pour déterminer une forme d'onde de neuromodulation spécifique au patient comprend la détermination d'au moins une valeur de paramètre de modulation de champ pour chaque segment de forme d'onde.

14. Support non transitoire lisible par machine selon l'une quelconque des revendications 8 à 13, dans lequel l'utilisation du fichier de forme d'onde et de l'emplacement cible du champ de neuromodulation pour déterminer une forme d'onde de neuromodulation spécifique au patient comprend :
la détermination d'une sensibilité du patient à la neuromodulation à l'emplacement cible de champ de neurostimulation ;
l'utilisation de la sensibilité du patient déterminée pour déterminer une plage de dose cible de neuromodulation ; et
la sélection d'au moins une valeur de paramètre de neuromodulation définie afin de fournir une dose spécifique au patient pour la forme d'onde de neuromodulation spécifique au patient qui se situe dans la plage de dose cible de neuromodulation.

15. Support non transitoire lisible par machine selon l'une quelconque des revendications 8 à 14, comprenant en outre des instructions qui, lorsqu'elles sont exécutées par une machine, amènent la machine à ajuster au moins une valeur de paramètre de modulation de champ de la forme d'onde et à ajuster l'au moins une valeur de paramètre de neuromodulation définie afin de maintenir des relations définies dans la forme d'onde lorsque l'au moins un paramètre de modulation de champ de la forme d'onde est ajusté.
